# EUROPÄISCHE PATENTSCHRIFT

(11) **EP 1 159 249 B1**
(45) Veröffentlichungstag und Bekanntmachung des Hinweises auf die Patenterteilung: **28.04.2004**
(21) Anmeldenummer: 00912517.0
(22) Anmeldetag: 28.02.2000
(51) Int. Cl.: C07C 57/07, B01D 5/00, B01D 3/16, B01D 3/26

(54) **FRAKTIONIERTE KONDENSATION EINES ACRYLSÄURE ENTHALTENDEN PRODUKTGASGEMISCHES**
FRACTIONAL CONDENSATION OF A PRODUCT GAS MIXTURE CONTAINING ACRYLIC ACID
CONDENSATION FRACTIONNEE D'UN MELANGE GAZEUX PRODUIT CONTENANT DE L'ACIDE ACRYLIQUE

(30) Priorität: 06.03.1999 DE 19909923; 28.05.1999 DE 19924532
(43) Veröffentlichungstag der Anmeldung: 05.12.2001
(73) Patentinhaber: BASF AKTIENGESELLSCHAFT, 67056 Ludwigshafen (DE)
(72) Erfinder: THIEL, Joachim, D-67435 Neustadt (DE); SCHRÖDER, Jürgen, D-67071 Ludwigshafen (DE); NESTLER, Gerhard, D-67061 Ludwigshafen (DE)
(86) Internationale Anmeldenummer: PCT/EP2000/001630
(87) Internationale Veröffentlichungsnummer: WO 2000/053561

(56) Entgegenhaltungen:
- WO-A-97/45184

## Beschreibung

Vorliegende Erfindung betrifft ein Verfahren der fraktionierten Kondensation eines Acrylsäure enthaltenden Produktgasgemisches einer heterogen katalysierten Gasphasen-Partialoxidation von C₃-Vorläufern der Acrylsäure mit molekularem Sauerstoff in einer hydraulisch abgedichtete Querstromböden als trennwirksame Einbauten enthaltenden Trennkolonne.

Acrylsäure ist ein bedeutendes Zwischenprodukt, das beispielsweise im Rahmen der Herstellung von Polymerisatdispersionen Verwendung findet.

Unter anderem ist Acrylsäure durch heterogen katalysierte Gasphasen-Partialoxidation von C₃-Vorläufern der Acrylsäure mit molekularem Sauerstoff an im festen Aggregatzustand befindlichen Katalysatoren bei erhöhter Temperatur erhältlich. Unter dem Begriff "C₃-Vorläufer" von Acrylsäure werden dabei solche chemischen Verbindungen zusammengefaßt, die formal durch Reduktion von Acrylsäure erhältlich sind. Bekannte C₃-Vorläufer von Acrylsäure sind z.B. Propan, Propen und Acrolein. Aber auch Verbindungen wie Propionaldehyd oder Propionsäure sind zu den C₃-Vorläufern zu zählen. Von ihnen ausgehend handelt es sich bei der heterogen katalysierten Gasphasen-Partialoxidation mit molekularem Sauerstoff wenigstens teilweise um eine oxidative Dehydrierung.

Bei den erfindungsrelevanten heterogen katalysierten Gasphasen-Partialoxidationen werden die genannten C₃-Vorläufer der Acrylsäure, in der Regel mit inerten Gasen wie z.B. Stickstoff, CO, CO₂, und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt.

Aus der DE-A 19740252, dem DE-A 19740253, der DE-A 19833049, der DE-A 19814375, der DE-A 19814421 und der DE-A 19814449 ist bekannt, daß aus Acrylsäure enthaltenden Produktgasgemischen von heterogen katalysierten Gasphasen-Partialoxidationen von C₃-Vorläufern der Acrylsäure eine Grundabtrennung der im Produktgasgemisch enthaltenen Acrylsäure dadurch möglich ist, daß man das Produktgasgemisch, gegebenenfalls nach direkter und/oder indirekter Vorkühlung, in einer mit trennwirksamen Einbauten versehenen Trennkolonne in sich selbst aufsteigend einer fraktionierten Kondensation unterwirft und die Acrylsäure über einen Seitenabzug der Trennkolonne als Rohacrylsäure entnimmt. Der Begriff Rohacrylsäure bringt dabei zum Ausdruck, daß es sich bei der über den Seitenabzug entnommenen Acrylsäure um kein reines Produkt, sondern um ein Gemisch handelt, das neben Acrylsäure (in der Regel ≥ 95 % des Gemischgewichtes) noch typische Nebenprodukte der Gasphasenoxidation (z.B. Wasser, niedere Aldehyde, Essigsäure, Propionsäure etc.) enthält.

Die WO 97/45184 offenbart eine Trennkolonne, die eine Kombination aus Regensiebböden und Querstromsiebböden aufweisen kann.

Gegenüber den sonstigen bekannten Verfahren zur Grundabtrennung einer Rohacrylsäure aus den Produktgasgemischen von heterogen katalysierten Gasphasen-Partialoxidationen von C₃-Vorläufern der Acrylsäure, die üblicherweise durch Aufnahme der Acrylsäure in ein geeignetes Absorptionsmittel und nachfolgende Entfernung des Absorptionsmittels über destillative Trennverfahren erfolgen, ist das vorstehend skizzierte Verfahren der fraktionierten Kondensation dadurch gekennzeichnet, daß es bei Zugabe von Polymerisationsinhibitoren in einem geringeren Umfang unerwünschte Polymerisatbildung bedingt.

Als trennwirksame Einbauten in den zur fraktionierten Kondensation des Produktgasgemisches der heterogen katalysierten Gasphasen-Partialoxidation von C₃-Vorläufern der Acrylsäure heranzuziehenden Trennkolonnen empfehlen die vorgenannten Schriften des Standes der Technik insbesondere Packungen, Füllkörper und/oder Böden, vorzugsweise Glockenböden, Siebböden, Ventilböden und/oder Dual-Flow-Böden. In den Ausführungsbeispielen werden ausschließlich Trennkolonnen verwendet, die entweder nur Glockenböden (hydraulisch abdichtende Querstromböden) oder nur Dual - Flow Böden als trennwirksame Einbauten enthalten. Nachteilig an den Empfehlungen des Standes der Technik ist, daß Packungen und Füllkörper unerwünschte Polymerisatbildung einerseits fördern und andererseits beim Auftreten von unerwünschter Polymerisatbildung vergleichsweise rasch ihre Durchgängigkeit verlieren. Von Nachteil ist ferner, daß bei alleiniger Verwendung von Dual-Flow-Böden als trennwirksame Einbauten die Trennleistung der Kolonne nicht voll zu befriedigen vermag. Bei einer alleinigen Verwendung von Glockenböden verliert die Trennkolonne ihre Durchgängigkeit ebenfalls nach nicht voll befriedigenden Standzeiten infolge Polymerisatbildung.

Die Aufgabe der vorliegende Erfindung bestand daher darin, ein Verfahren der fraktionierten Kondensation eines Acrylsäure enthaltenden Produktgasgemisches einer heterogen katalysierten Gasphasen-Partialoxidation von C₃-Vorläufern der Acrylsäure mit molekularem Sauerstoff in einer hydraulischen abgedichtete Querstromböden als trennwirksame Einbauten enthaltenden Trennkolonne zur Verfügung zu stellen, das die Nachteile der genannten Verfahren des Standes der Technik mindert.

Demgemäß wurde ein Verfahren der fraktionierten Kondensation eines Acrylsäure enthaltenden Produktgasgemisches einer heterogen katalysierten Gasphasen-Partialoxidation von C₃-Vorläufern der Acrylsäure mit molekularem Sauerstoff in einer hydraulisch abgedichtete Querstromböden als trennwirksame Einbauten enthaltenden Trennkolonne gefunden, das dadurch gekennzeichnet ist, daß als Trennkolonne eine solche verwendet wird, die von unten nach oben zunächst Dual-Flow-Böden und im Anschluß daran hydraulisch abgedichtete Querstromböden als trennwirksame Einbauten enthält.

Unter Dual-Flow-Böden werden in dieser Schrift Platten mit einfachen Durchtrittsstellen (Löcher, Schlitze etc.) verstanden. Das in der Kolonne aufsteigende Gas und die in der Kolonne absteigende Rücklaufflüssigkeit treten entgegengesetzt strömend durch die gleichen Durchtrittsstellen. Der Querschnitt der Durchtrittsstellen wird in an sich bekannter Weise der Belastung der Kolonne angepaßt. Ist er zu klein, strömt das aufsteigende Gas mit so hoher Geschwindigkeit durch die Durchtrittsstellen, daß die in der Kolonne absteigende Rücklaufflüssigkeit im wesentlichen ohne Trennwirkung mitgerissen wird. Ist der Querschnitt der Durchtrittsstellen zu groß, bewegen sich aufsteigendes Gas und absteigender Rücklauf im wesentlichen ohne Austausch aneinander vorbei und der Boden läuft Gefahr trocken zu laufen. Üblicherweise weisen Dual-Flow-Böden kein Ablaufrohr auf, das sie mit dem nächsten Boden verbindet. Selbstredend schließt jeder Dual-Flow Boden bündig mit den Kolonnenwänden ab. Mit abnehmender Belastung der Kolonne laufen Dual-Flow Böden trocken. Hydraulisch abgedichtete Querstromböden sind dadurch charakterisiert, daß sie beim Abschalten der Kolonne nicht leerlaufen können, sieht man einmal von der winzigen Leerlaufbohrung (ihr Querschnitt ist normalerweise mehr als 200 mal kleiner als der Gesamtquerschnitt der Durchtrittsstellen) ab, die jeder Querstromboden aus Zweckmäßigkeitsgründen aufweist. D.h., auch bei geringen Kolonnenbelastungen weisen hydraulisch abgedichtete Querstromböden gestaute Rücklaufflüssigkeit auf und laufen keine Gefahr, trocken zu laufen. Dies ist dadurch bedingt, daß es sich bei den Durchtrittsstellen von hydraulisch abgedichteten Querstromböden nicht wie bei den Dual-Flow Böden um kaminlose Bohrungen handelt.

Vielmehr mündet jede Durchtrittsstelle in einen Kamin, der ein Trockenlaufen unterbindet. Über dem Kamin sind Dampfumlenkhauben (Glocken) angebracht, die in die gestaute Bodenflüssigkeit eintauchen. Häufig sind die Dampfumlenkhauben an ihren Rändern geschlitzt oder gezackt (d.h., sie weisen Treibschlitze auf). Der durch die Durchtrittsstelle aufsteigende Dampfstrom erfährt durch die Dampfumlenkhauben eine Ablenkung und strömt parallel zum Boden, d.h., quer zur Kolonne, in die gestaute Flüssigkeit. Die aus benachbarten Glocken austretenden Dampfblasen treffen aufeinander und bilden eine Sprudelschicht aus. Ablaufrohre bzw. -segmente, die, in der Regel abwechselnd links oder rechts, die Böden verlassen, regeln - von Wehren unterstützt - den Flüssigkeitsstand der Böden und führen den Rücklauf dem darunterliegenden Boden zu. Für die hydraulisch abdichtende Wirkung ist wesentlich, daß die Ablaufrohre bzw.-segmente des oberen Bodens in die gestaute Flüssigkeit des darunter liegenden Bodens tauchen. Vorzugsweise sind keine Zulaufwehre vorhanden. In der Höhe einstellbare Glocken gestatten ein Anpassen an die Strömungsverhältnisse und den Ausgleich der Eintauchtiefen bei Herstellungsungleichmäßigkeiten, so daß alle Glocken des Bodens gleichmäßig gasen. Auf dem Boden angebrachte Leitbleche leiten bei Bedarf die Flüssigkeit in vorgeschriebenen Bahnen und schaffen für den Bodenwirkungsgrad günstige relative Strömungen zwischen Dampf und Flüssigkeit.

Je nach Gestalt und Anordnung der Glocken unterscheidet man z.B. Rundglockenböden (Durchtrittsstelle, Kamin und Glocke sind rund), Thormann Böden (Durchtrittsstelle, Kamin und Glocke sind rechtekkig, die Böden sind hintereinander angeordnet, wobei die längere Rechteckkante senkrecht zur Flüssigkeitsströmung weist) und Tunnel Böden (wie Thormann Böden, die längere Rechteckkante ist jedoch parallel zur Flüssigkeitsströmung ausgerichtet).

In typischer Weise ist das Acrylsäure enthaltende Produktgasgemisch einer heterogen katalysierten Gasphasen-Partialoxidation von C₃-Vorläufern der Acrylsäure mit molekularem Sauerstoff an im festen Aggregatzustand befindlichen Katalysatoren wie folgt zusammengesetzt:
1 bis 30 Gew.-% Acrylsäure,
0,05 bis 10 Gew.-% molekularer Sauerstoff,
1 bis 30 Gew.-% Wasser,
< 5 Gew.-% Essigsäure,
< 3 Gew.-% Propionsäure,
< 1 Gew.-% Maleinsäure und/oder Maleinsäure-Anhydrid,
< 2 Gew.-% Acrolein,
< 1 Gew.-% Formaldehyd,
< 1 Gew.-% Furfural,
< 0,5 Gew.-% Benzaldehyd,
< 1 Gew.-% Propen und als
Restmenge inerte Gase wie z.B. Stickstoff, Kohlenmonoxid, Kohlendioxid, Methan oder Propan.

Die Gasphasenoxidation selbst kann wie im Stand der Technik beschrieben durchgeführt werden. Ausgehend von Propan kann die Gasphasenoxidation z.B. in zwei aufeinanderfolgenden Oxidationsstufen durchgeführt werden, wie sie in der EP-A 700714 und in der EP-A 700893 beschrieben sind. Selbstverständlich können aber auch die in der DE-A 19 740 253 sowie in der DE-A 19 740 252 zitierten Gasphasenoxidationen zur Anwendung kommen.

In der Regel beträgt die Temperatur des die Gasphasenoxidation verlassenden Produktgasgemisches 150 bis 350°C, häufig 200 bis 300°C. In einem Quenchsystem 1 wird das heiße Produktgasgemisch zweckmäßigerweise zunächst durch direkte Kühlung auf eine Temperatur von 100 bis 180°C abgekühlt, bevor es zum Zweck der fraktionierten Kondensation in den untersten Abschnitt der erfindungsgemäß zu verwendenden Trennkolonne geführt wird. Der in der Kolonne herrschende Betriebsdruck beträgt in der Regel 0,5 bis 5 bar, häufig 0,5 bis 3 bar und vielfach 0,5 bis 2 bar.

Als Quenchvorrichtung 1 können alle im Stand der Technik für diesen Zweck bekannten Vorrichtungen (z.B. Sprühwäscher, Venturiwäscher, Blasensäulen oder sonstige Apparate mit berieselten Oberflächen) eingesetzt werden, wobei vorzugsweise Venturi-Wäscher oder Sprühkühler verwendet werden.

Als Quenchflüssigkeit 1 kann z.B. Sumpfflüssigkeit aus der erfindungsgemäßen fraktionierten Kondensation verwendet werden. Zweckmäßigerweise wird beim Quenchen nicht verdampfte Quenchflüssigkeit 1, gegebenenfalls über einen indirekt kühlenden Wärmetauscher, im Kreis geführt. Ein Teil der im Kreis geführten Quenchflüssigkeit wird als Schwersiederauslaß in sinnvoller Weise kontinuierlich ausgeschleust. Bei Bedarf können die im Ausgeschleusten enthaltenen, durch Michael-Addition in reversibler Weise gebildeten Acrylsäureoligomere durch Einwirkung erhöhter Temperaturen (130 bis 250°C) und gegebenenfalls unter Zugabe saurer bzw. basischer Spaltkatalysatoren bei reduziertem Druck in Acrylsäure rückgespalten werden. Die dabei dampfförmig entweichende Acrylsäure wird in zweckmäßigerweise kondensiert und in den Kreislauf der Quenchflüssigkeit 1 rückgeführt.

Die Einleitstelle des gequenchten Produktgasgemisches der katalytischen Gasphasenoxidation in die erfindungsgemäß zu verwendende Trennkolonne befindet sich zweckmäßigerweise unterhalb des untersten Dual-Flow-Bodens. Prinzipiell kann die fraktionierte Kondensation innerhalb der Kolonne in an sich bekannter Weise durch indirekte Kühlung und/oder Erwärmung bewirkt werden. Es ist jedoch zweckmäßig die fraktionierte Kondensation wie folgt zu bewirken.

Über einen oberhalb der Einleitsstelle und unterhalb des untersten Dual-Flow-Boden angebrachten ersten Fangboden wird ein Teil der beim Aufsteigen des gequenchten Produktgasgemisches sich bildenden und/oder kondensierenden, schwerer als Acrylsäure flüchtigen, Substanzen entnommen. Ein Teil der entnommenen Schwersiedefraktion kann im Gemisch mit der Kolonne entnommener Sumpfflüssigkeit als Quenchflüssigkeit 1 verwendet werden. Die dabei verbleibende Teilmenge der entnommenen Schwersiederfraktion wird in einem indirekten Wärmetauscher abgekühlt oder erwärmt und oberhalb der ersten, aber unterhalb eines in der unteren Kolonnenhälfte angebrachten zweiten Fangbodens in die Kolonne rückgeführt. Über den zweiten Fangboden wird im Seitenabzug als Mittelsiederfraktion die Rohacrylsäure entnommen, die im Normalfall eine Reinheit ≥95 Gew.-% aufweist. Zweckmäßigerweise wird man die Rohacrylsäure weiteren destillativen und/oder kristallisativen Weiterreinigungsstufen zuführen und wenigstens einen Teil der im Rahmen der Destillationen und/oder Kristallisationen anfallenden Sumpfflüssigkeiten und/oder Mutterlaugen unterhalb des zweiten, aber oberhalb des ersten Fangbodens in die Kolonne rückführen. Alternativ dazu kann die Rückführung der Sumpfflüssigkeiten und/oder Mutterlaugen auch so erfolgen, daß sie in die zwei Teilströme aufgeteilt werden, von denen einer unterhalb des zweiten, aber oberhalb des ersten Fangbodens und der andere oberhalb des zweiten Fangbodens in die Kolonne rückgeführt wird. Der letztgenannte der beiden Teilströme wird in der Regel, bezogen auf die Gesamtrückführung, bis zu 35 Gew.-% betragen. Aus dem am Kopf der Kolonne entweichenden Leichtsiedergasstrom wird man zweckmäßig durch direkte Kühlung in einem an Einbauten freien oder Einbauten enthaltenden Raum mittels einer zweiten Quenchflüssigkeit (in dieser Schrift zum Zweck der Differenzierung als Quenchflüssigkeit 2 bezeichnet) im wesentlichen Wasser sowie schwerer als Wasser flüchtige Bestandteile auskondensieren. Das dabei gewonnene Kondensat wird als Sauerwasser bezeichnet. Einen Teil des Sauerwassers wird man in sinnvoller Weise zur Erhöhung der Trennleistung am Kopf der Kolonne in selbige rückführen. Ein weiterer Teil des Sauerwassers wird zweckmäßigerweise ausgeschleust und entsorgt (z.B. verbrannt) und der verbleibende Teil des Sauerwassers wird üblicherweise in einem externen Wärmetauscher indirekt abgekühlt und als Quenchflüssigkeit 2 verwendet.

Leichter als Wasser flüchtige Bestandteile des Leichtsiederstroms werden normalerweise im wesentlichen gasförmig abgezogen und gegebenenfalls als Verdünnungsgas in die Gasphasenoxidation rückgeführt.

Alternativ kann man den Sauerwasserquench in die Kolonne für die fraktionierte Kondensation integrieren. In diesem Fall wird über einen weiteren Fangboden im oberen Teil der Kolonne wäßrige Rücklaufflüssigkeit entnommen, in einem Wärmetauscher indirekt gekühlt und bis auf den zu entsorgenden Auslaßanteil teilweise am Kopf der Kolonne und teilweise unterhalb des Fangbodens rückgeführt. Das gegebenenfalls in die Gasphasenoxidation rückzuführende Abgas verläßt die Kolonne in diesem Fall an deren Kopf.

Zweckmäßigerweise sollten sich beim erfindungsgemäßen Verfahren die Dual-Flow-Boden in der Trennkolonne wenigstens bis zum Seitenabzug der Rohacrylsäure erstrecken. Vorzugsweise erstrecken sich die Dual-Flow-Böden beim erfindungsgemäßen Verfahren in der Trennkolonne aber in etwa bis zu dem Querschnitt in der Trennkolonne, von dem ab die Acrylsäuregehalte der Rücklaufflüssigkeit zum Kolonnenkopf hin betrachtet ≤ 20 Gew.-%, bezogen auf das Gewicht der Rücklaufflüssigkeit, betragen. Die Anzahl an Dual-Flow-Böden beträgt für das erfindungsgemäße Trennverfahren in der Regel 5 bis 60, bevorzugt 20 bis 40. Das Öffnungsverhältnis der Dual-Flow-Böden liegt ferner zweckmäßig bei 10 bis 25 %, bevorzugt bei 12 bis 20 %. Als Durchtrittstellen weisen die erfindungsgemäß einzusetzenden Dual-Flow-Böden üblicherweise kreisrunde Löcher auf, deren Lochdurchmesser normalerweise 5 bis 50 mm, häufig 10 bis 20 mm betragen. Häufig wird man in der Trennkolonne von unten nach oben die Lochdurchmesser verjüngen und/oder die Anzahl der Löcher vermindern. Sinnvoll ist es, wenn die Anzahl der eingesetzten Dual-Flow-Böden für das erfindungsgemäße Verfahren etwa 8 bis 20, häufig etwa 10 bis 15 theoretischen Trennstufen entspricht.

Die Anzahl der sich an die Dual-Flow-Böden anschließenden hydraulisch abgedichteten Querstromböden wird in der Regel 5 bis 60, häufig 15 bis 40 betragen. Zweckmäßigerweise weisen diese Böden Treibschlitze zur besseren Zwangsführung der Flüssigkeit über den Boden auf. Das Öffnungsverhältnis der erfindungsgemäß zu verwendenden Querstromböden wird in der Regel 5 bis 25 %, häufig 10 bis 20 % betragen (das Öffnungsverhältnis gibt den prozentualen Anteil der Durchtrittsquerschnitte am Gesamtquerschnitt wieder). In der Regel wird die Anzahl der hydraulisch abgedichteten Querstromböden für das erfindungsgemäße Verfahren so bemessen, daß sie etwa 10 bis 30, häufig ca. 15 bis 25 theoretischen Trennstufen entspricht. Vorzugsweise werden für das erfindungsgemäße Verfahren Thormann-Böden verwendet. Wird der Sauerwasserquench in die Kolonne für die fraktionierte Kondensation integriert, so kommen für diesen Bereich der Trennkolonne (Acrylsäuregehalt der Rücklaufflüssigkeit von unten nach oben betrachtet in der Regel ≤ 10 Gew.-%) als trennwirksame Einbauten für das erfindungsgemäße Verfahren prinzipiell alle aus der Rektifikationstechnik bekannten Einbauten-Typen in Betracht. D.h., es können in diesem Bereich der Trennkolonne sowohl Quer- als auch Gegenstromböden, aber auch Füllkörper und strukturierte Packungen verwendet werden. Die bevorzugte Ausführungsform sind als Ventilböden ausgeführte Querstromböden. Ventilböden besitzen z.B. Bodenbohrungen mit hubbegrenzten Teller-, Ballast- oder Hebeventilen (Schwimmklappen), die die Größe der Dampfdurchtrittsöffnung der jeweiligen Kolonnenbelastung anpassen. Der aufsteigende Dampfstrom wird abgelenkt, strömt parallel zum Boden in die gestaute Rücklaufflüssigkeit und bildet eine Sprudelschicht aus. Bewehrte Ablaufrohre führen den Rücklauf von Boden zu Boden. Die Dampfgeschwindigkeit darf eine konstruktionsbedingte untere Belastungsgrenze nicht unterschreiten, sonst fließt die Bodenflüssigkeit ab.

Die Polymerisationsinhibierung des erfindungsgemäßen Verfahrens kann im wesentlichen wie in der DE-A 19909923 beschrieben durchgeführt werden. D.h., sie kann beispielsweise dadurch erfolgen, daß am Kopf der Kondensationskolonne Phenothiazin oder ein Gemisch aus einem N-Oxyl-Radikal und einer Benzolverbindung, die zwei über Heteroatome an den aromatischen Ringe gebundene Substituenten und wenigstens einen beweglichen Wasserstoff aufweist, zugesetzt wird.

Eine günstigere Form der Polymerisationsinhibierung ist dadurch gekennzeichnet, daß sie ausschließlich mittels N-Oxyl-Radikalen (z.B. die in der EP-A 765 956 genannten) erfolgt. Das sind Verbindungen, die wenigstens eine Gruppe -N-O• aufweisen.

Erfindungsgemäß bevorzugte N-Oxyl-Radikale sind die Pyrrolidin-1-oxyl-Typen und die Piperidin-1-oxyl-Typen. Beispielhaft genannt seien 4,4',4''-Tris-(2,2,6,6-tetramethylpiperidin-1-oxyl)phosphit, 2,2,6,6-Tetramethylpiperidin-1-oxyl (TEMPO), 4-Hydroxy-2,2,6,6-tetramethylpiperidin-1-oxyl (4-OH-TEMPO), 4-Oxo-2,2,6,6-tetramethylpiperidin-1-oxyl (4-Oxo-TEMPO), 4-Dimethylamino-2,2,6,6,-tetramethylpiperidin-1-oxyl, 4-Amino-2,2,6,6-tetramethylpiperidin-1-oxyl, 4-Ethanoyloxy-2,2,6,6-tetramethylpiperidin-1-oxyl, 2,2,5,5-Tetramethylpyrrolidin-1-oxyl und 3-Amino-2,2,5,5,-tetramethylpyrrolidin-1-oxyl. Die N-Oxyl-Inhibitoren werden vorzugsweise als 0,1 bis 2 gew.-%ige Lösungen in Wasser und/oder in Sauerwasser eingesetzt. Die Zugabe der wäßrigen N-Oxyl-Inhibitorlösung erfolgt zweckmäßigerweise im oberen Viertel der Kondensationskolonne und/oder in den Sauerwasserquench. Die wäßrige Lösung kann nur ein N-Oxyl-Radikal oder ein Gemisch von N-Oxyl-Radikalen enthalten. Eine ausreichende Inhibierung kann bereits durch Zugabe einer wäßrigen Lösung erzielt werden, die ausschließlich 4-OH-TEMPO als Polymerisationsinhibitor enthält.

Die Zugabemenge der einzusetzenden N-Oxyl-Inhibitoren wird in zweckmäßiger Weise so bemessen, daß die aus der Kolonne entnommene Schwersiederfraktion und Sumpfflüssigkeit 1 bis 1000 Gew.-ppm, bezogen auf das Gewicht der Schwersiederfraktion, an N-Oxyl-Inhibitoren enthält. Da die aus der Kolonne entnommene Schwersiederfraktion und Sumpfflüssigkeit als Quenchflüssigkeit 1 dient, wird das Quenchsystem 1 automatisch mitstabilisiert. Bei Bedarf kann das Quenchsystem 1 durch Zusatz einer Phenothiazinverbindung co-stabilisiert werden. Als solche Phenothiazinverbindungen kommen beispielsweise Phenothiazin (PTZ) selbst, Bis-(α-methylbenzyl)phenothiazin, 3,7-Dioctylphenothiazin und Bis-(α-dimethylbenzyl)phenothiazin in Betracht, unter denen das Phenothiazin bevorzugt ist. Letzteres gilt insbesondere dann, wenn 4-OH-TEMPO zur Stabilisierung der Kondensationskolonne mitverwendet oder ausschließlich verwendet wird. Ein solcher Phenothiazinzusatz kann, bezogen auf das Gewicht der Quenchflüssigkeit 1, 1 bis 500 Gew.-ppm betragen. Zweckmäßigerweise erfolgt ein solcher Zusatz einer Phenothiazinverbindung in Acrylsäure, vorzugsweise in über Seitenabzug entnommener Rohacrylsäure gelöst (typisch 0,1 bis 2 gew.-%ig).

Der zur Polymerisationsinhibierung erforderliche Bedarf an N-Oxyl-Radikalen läßt sich dadurch absenken, daß die weniger anspruchsvolle Sauerwasserstabilisierung, d.h., die Stabilisierung des Quenchsystems 2, ersatzweise oder in Kombination mit einer wäßrigen Lösung wenigstens einer Benzolverbindung, die zwei über Heteroatome an den aromatischen Ring gebundene Substituenten und wenigstens einen beweglichen Wasserstoff aufweist (z.B. die in der EP-A 765 856 genannten), z.B. einer Phenolverbindung, durchgeführt wird (typisch 0,1 bis 2 gew.-%ige Lösung). Als solche Phenolverbindungen kommen beispielsweise Hydrochinon oder Methochinon (p-Methoxyphenyl = MEHQ) in Betracht, unter denen letztere bevorzugt ist. Dies gilt insbesondere dann, wenn im Kopfbereich der Kolonne ausschließlich 4-OH-TEMPO eingesetzt und das Quenchsystem mit PTZ co-stabilisiert wird. In der Regel werden, bezogen auf das Gewicht des Sauerwassers, 1 bis 500 Gew.-ppm wenigstens einer Phenolverbindung für eine solche Sauerwasserinhibierung verwendet.

Eine alternative Polymerisationsinhibierung besteht darin, am Kopf der Kondensationskolonne eine wäßrige Lösung von MEHQ aufzugeben und die Sauerwasserinhibierung ebenfalls durch Zusatz einer Lösung von MEHQ in Wasser und/oder in Sauerwasser durchzuführen. Zusätzlich wird im Mittelteil der Kondensationskolonne sowie gegebenenfalls im Produktgasgemischquench (Quench 1) eine Lösung von PTZ in Acrylsäure (z.B. Rohacrylsäure) aufgegeben.

Wie bereits erwähnt, wird das Quenchsystem 1 über die Stabilisierung der Kondensationskolonne automatisch mitstabilisiert und kann bei Bedarf durch Zusatz von Phenothiazin und/oder Metochinon co-stabisisiert werden.

Die dabei erzielte Polymerisationsinhibierung der Quenchflüssigkeit 1 ist in der Regel auch ausreichend, um bei einer in das erfindungsgemäße Verfahren integrierten Rückspaltung eine ausreichende Stabilität des Auslaß der Quenchflüssigkeit gegen unerwünschte radikalische Polymerisatbildung zu gewährleisten. Hingegen werden bei der Kondensation der im Rahmen der Rückspaltung gasförmig entweichenden, Acrylsäure enthaltenden, Dämpfe die Kondensatoroberflächen zweckmäßigerweise extra inhibiert (die in der Quenchflüssigkeit 1 enthaltenen Inhibitoren verdampfen in der Regel nicht mit). Diese Polymerisationsinhibierung der Kondensatoroberflächen wird man mit Vorteil mit denselben Polymerisationsinhibibitoren vornehmen, die auch zur Inhibierung der Kondensationskolonne verwendet und/oder empfohlen wurden.

Verwendet man zur Stabilisierung der Kondensationskolonne und des Sauerwasserquench z.B. ausschließlich 4-OH-TEMPO, so ist es sinnvoll, die Kondensatoroberflächen auch ausschließlich mit 4-OH-TEMPO, zweckmäßig in Rohacrylsäure gelöst, vorzunehmen. Selbstverständlich können die Kondensatoroberflächen aber auch mittels PTZ, MEHQ und/oder Hydrochinon co-stabilisiert oder ausschließlich stabilisiert werden.

In der Regel wird eine integrierte Rückspaltung bei einem Druck von ≤ 1 bar und bei einer Temperatur von 130 bis 250°C durchgeführt.

Mit Vorteil beträgt der Druck für die Rückspaltung 25 bis 600, vorzugsweise 100 bis 300 mbar. Die Rückspalttemperatur liegt zweckmäßigerweise bei 140 bis 230°C, vorzugsweise bei 160 bis 200°C. Wird die Rückspaltung kontinuierlich durchgeführt (das erfindungsgemäße Verfahren wird vorzugsweise kontinuierlich durchgeführt), so sollte die Verweilzeit im Rückspaltreaktor etwa 0,5 bis 3 h betragen. In einfacher Weise läßt sich die erfindungsgemäß zu integrierende Rückspaltung in einem beheizbaren Rührreaktor durchführen. Wie in der US-A 57 33 075 sowie in der DE-A 41 01 879 beschrieben, läßt sich die Rückspaltung der im Auslaß des Quenchsystems 1 enthaltenen Acrylsäureoligomere ohne Zusatz spezieller saurer oder basischer Spaltkatalysatoren durchführen. Mit Vorteil wird man die Rückspaltung jedoch im Beisein von Spaltkatalysatoren durchführen. Als solche kommen z.B. Dodecylbenzolsulfonsäure, p-Toluolsulfonsäure, Schwefelsäure oder die festen sauren Katalysatoren der JP-A 3-178949 in Betracht.

Insbesondere im Fall einer Polymerisationsinhibierung mittels N-Oxyl-Radikalen, vor allem wenn 4-OH-TEMPO als alleiniger Polymerisationsinhibitor oder als co-Stabilisator des Quenchsystems 1 verwendet wird, ist es zweckmäßig die Rückspaltung durch Zusatz eines anorganischen Salzes, dessen Zusatz zu einer wäßrigen Lösung einer starken Brönsted-Säure den pH-Wert der wäßrigen Lösung ins alkalische verschiebt, vorzunehmen, wie es z.B. die DE-C 2407236 empfiehlt. Bezogen auf die der Rückspaltung zu unterwerfende Menge an Auslaß der Quenchflüssigkeit 1 wird die zu zusetzende Menge an basischem Rückspaltkatalysator in der Regel 0,1 bis 5 Gew.-% betragen. Beispiele für erfindungsgemäß geeignete Rückspaltkatalysatoren sind KOH, K₂CO₃, KHCO₃, NaOH, Na₂CO₃, NaHCO₃, LiOH, Li₂CO₃ und CaCO₃. D.h., geeignete Rückspaltkatalysatoren sind insbesondere die Alkali- und/oder Erdalkalisalze von schwachen anorganischen oder organischen Brönstedsäuren wie z.B. Phosphorsäure, Borsäure, Kohlensäure, Ameisensäure oder Essigsäure.

Mit anderen Worten eignen sich somit als Rückspaltkatalysatoren vor allem Alkali- und/oder Erdalkaliphosphate, -borate, -carbonate, -hydrogencarbonate, -formiate und -acetate.

Vorzugsweise wird man die Rückspaltkatalysatoren so wählen, daß sie unter den Rückspaltbedingungen im Auslaß der Quenchflüssigkeit 1 löslich sind. Gemäß US-A 4293347 wirkt sich auch ein Beisein von Dialkylphthalaten vorteilhaft auf die relevante Rückspaltung aus.

Der im Spaltreaktor verbleibende schwerflüchtige Rückstand wird beim erfindungsgemäßen Verfahren regelmäßig seiner Entsorgung, z.B. seiner Verbrennung, zugeführt.

An dieser Stelle sei noch festgehalten, daß der Quenchflüssigkeit 1 bei Bedarf eine höher als Acrylsäure siedende inerte organische Flüssigkeit zugesetzt werden kann, die die Quenchflüssigkeit 1 fluid hält. Als solche hochsiedenden inerten organischen Flüssigkeiten kommen insbesondere alle diejenigen in Betracht, die in der DE-A 21 36 396 und in der DE-A 43 08 087 empfohlen werden. Dies sind im wesentlichen Flüssigkeiten, deren Siedepunkt bei Normaldruck oberhalb von 160°C liegt. Beispielhaft genannt seien Ethylhexansäure, N-Methylpyrrolidon, Mittelölfraktionen aus der Paraffindestillation, Diphenylether, Diphenyl oder Mischungen der vorgenannten Flüssigkeiten wie z.B. ein Gemisch aus 70 bis 75 Gew.-% Diphenylether und 25 bis 30 Gew.-% Diphenyl. Günstig ist die Verwendung eines Gemisches bestehend aus einer Mischung aus 70 bis 75 Gew.-% Diphenylether und 25 bis 30 Gew.-% Diphenyl, sowie, bezogen auf diese Mischung 0,1 bis 25 Gew.-% o-Dimethylphthalat.

Im vorgenannten Fall wird bei der Rückspaltung wenigstens eine Teilmenge der mitverwendeten inerten organischen Flüssigkeit mitverdampfen. Verbleibt eine Teilmenge der organischen Flüssigkeit im Spaltrückstand, kann selbiger einer Aufarbeitung zugeführt werden, in der das mitverwendete Lösungsmittel, z.B. destillativ, abgetrennt und in den Quench 1 rückgeführt wird. Die verbleibenden Schwersieder werden entsorgt.

Als Quenchvorrichtung 1 können alle im Stand der Technik für diesen Zweck bekannten Vorrichtungen (z.B. Sprühwäscher, Venturiwäscher, Blasensäulen oder sonstige Apparate mit berieselten Oberflächen) eingesetzt werden, wobei erfindungsgemäß vorzugsweise Venturi-Wäscher oder Sprühkühler verwendet werden.

Zur indirekten Kühlung oder Erwärmung der Quenchflüssigkeit 1 eignen sich alle gängigen Wärmeüberträger oder Wärmetauscher. Als bevorzugt seien Rohrbündelwärmetauscher, Plattenwärmetauscher und Luftkühler genannt. Die Temperatur der Quenchflüssigkeit 1 beträgt nach Verlassen des Wärmetauschers normalerweise 70 bis 200°C, häufig 100 bis 150°C. Geeignete Kühlmedien sind Luft beim entsprechenden Luftkühler und Kühlflüssigkeiten, insbesondere Wasser, bei den anderen Kühlvorrichtungen. Die im Seitenabzug entnommene Rohacrylsäure enthält in der Regel noch

| | |
|---|---|
| 0,1 bis 2 Gew.-% | Essigsäure, |
| 0,5 bis 5 Gew.-% | Wasser, |
| 0,05 bis 1 Gew.-% | niedermolekulare Aldehyde, |
| 0,001 bis 1 Gew.-% | Maleinsäure und/oder deren Anhydrid sowie |
| 1 bis 500 Gew.ppm | Polymerisationsinhibitor, |

jeweils bezogen auf das Gewicht der Rohacrylsäure. Die Temperatur im Sumpf der Kolonne liegt in typischer Weise bei 90 bis 130°C, wohingegen die Kopftemperatur normalerweise 40 bis 80, häufig 50 bis 70°C beträgt. Die Entnahmetemperatur der Rohacrylsäure liegt meist bei 80 bis 110°C. Die Rückführtemperatur der Schwersieder beträgt beim Eintritt in die Kolonne in typischer Weise 95 bis 115°C. Die Rückführtemperatur des Sauerwassers in die Kolonne liegt in der Regel bei 25 bis 35°C. Prinzipiell kann das Quenchsystem 2 wie das Quenchsystem 1 gestaltet werden. Selbstverständlich kann erfindungsgemäß die Entnahme der Rohacrylsäure auch über mehrere in kurzen Abständen aufeinanderfolgende Fangböden vorgenommen werden.

Die als Mittelsiederfraktion entnommene Rohacrylsäure kann zum Zwecke der Weiterreinigung z.B. einer Kristallisation zugeführt werden. Hierbei wird in der Regel ohne Zusatz eines Lösungsmittels, insbesondere ohne Zusatz eines organischen Lösungsmittels gearbeitet. Das zu verwendende Kristallisationsverfahren unterliegt keiner Beschränkung. Die Kristallisation kann kontinuierlich oder diskontinuierlich, einstufig oder mehrstufig bis zu nahezu beliebigen Reinheitsgraden durchgeführt werden. Bei Bedarf kann der kristallisativ zu reinigenden Rohacrylsäure vorab einer Kristallisation Wasser zugesetzt werden (bezogen auf die enthaltene Menge an Acrylsäure bis zu 10 Gew.-% oder mehr, vorzugsweise bis zu 5 Gew.-%). Ein solcher Zusatz erleichtert die Abtrennung von in der Rohacrylsäure als Nebenprodukt enthaltener Essigsäure, da diese im Beisein von Wasser in geringerem Ausmaß in die Acrylsäurekristalle eingebaut wird. Außerdem mindert ein Beisein von Wasser die Verkrustungsneigung.

Es überrascht, daß eine veresterungsgerechte Rohacrylsäure bereits durch eine einzige Kristallisationsstufe erzielt werden kann. Zweckmäßigerweise wird diese Kristallisationsstufe als Suspensionskristallisation ausgeführt. Als Kristaller wird dazu vorteilhaft ein Trog verwendet, in welchem gewischte Kühlplatten (die innerlich von einem Kühlmedium durchflossen werden) hintereinander hängend angeordent sind. Durch das Wischen der Kühlplatten wird die Ausbildung einer Schichtkristallisation unterdrückt. Die Rohacrylsäure wird von hinten nach vorne kontinuierlich durch den Kristaller geführt (gepumpt oder überlaufgeregelt). Die einphasige Rohacrylsäure verdickt dabei zu einer zweiphasigen, Acrylsäurekristalle als feste Phase enthaltenden, Suspension. Ein diesbezüglich besonders geeigneter Kristaller ist ein Kristaller der Fa. GMF Gouda (Holland) vom Typ Kühlscheibenkristallisator (Cooling Disc Crystallizer). In zweckmäßiger Weise werden die gebildeten Kristalle aus der vorgenannten Suspension mittels einer Zentrifuge abgetrennt (z.B. eine solche der Fa. Siebtechnik, vom Typ Schubzentrifuge SHS mit konischer Siebtrommel) und bei Bedarf mit bereits weiter gereinigter Rohacrylsäure gewaschen und/oder einem später beschriebenen Schwitzen unterworfen. Das Abtrennen und Waschen der Suspensionskristalle kann aber auch vorteilhaft in einer Waschkolonne durchgeführt werden, wie es z.B. in der EP-A 97405, US-A 3872009, EP-A 98637, EP-A 305316, EP-A 105524 und der WO 84/00118 beschrieben ist. Dann werden die Kristalle üblicherweise in einen Behälter gegeben, der vorteilhaft bereits eine Menge aufgeschmolzener, in entsprechender Weise gereinigter, Acrylsäurekristalle enthält. Bei Bedarf enthält diese aufgeschmolzene Acrylsäure zusätzlichen Polymerisationsinhibitor zugesetzt (z.B. MEHQ, PTZ oder 4-Hydroxy-TEMPO). In der Regel genügt jedoch der in den Kristallen verbliebene Inhibitorrest, um eine ausreichende Inhibierung zu gewährleisten. Durch indirektes Erwärmen werden anschließend die gewonnenen Acrylsäurekristalle aufgeschmolzen. Die so erhältliche Acrylsäureschmelze weist normalerweise eine Reinheit ≥98 Gew.-% auf und kann unmittelbar als veresterungsgerechte Acrylsäure vermarktet werden.

Anstelle einer Suspensionskristallisation kann auch eine Schichtkristallisation, z.B. eine Fallfilmkristallisation, angewendet werden, wie sie z.B. in der EP-A 616 998. zur Gewinnung von Reinacrylsäure beschrieben ist. Als flüssiger Kälte-/Wärmeträger kommen dabei z.B. Wasser/Methanol, Wasser/Ethanol- und Wasser/Ethylenglycolmischungen in Betracht.

Zur Gewinnung besonders hoher Reinheitsgrade ("Reinacrylsäure") wird die Kristallisation zweckmäßigerweise als fraktionierte Kristallisation durchgeführt. Üblicherweise werden bei fraktionierter Kristallisation alle Stufen, die ein Kristallisat erzeugen, das reiner ist als die zugeführte Schmelze, Reinigungsstufen genannt. Alle anderen Stufen heißen Abtriebsstufen. Zweckmäßigerweise werden mehrstufige Verfahren nach dem Gegenstromprinzip betrieben, bei dem nach der Kristallisation in jeder Stufe das Kristallisat von der Mutterlauge abgetrennt und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung während der Kristallisation zwischen +5 und + 14°C, insbesondere zwischen +8°C und +12°C. Der Feststoffgehalt im Kristallisator liegt vorteilhafterweise zwischen 0 und 80 g Feststoff/100 g Gesamtmasse. Bei der Suspensionskristallisation beträgt der Feststoffgehalt vorzugsweise zwischen 15 und 35 g Feststoff/100 g Gesamtmasse und bei der Schichtkristallisation 50 bis 80 g Feststoff/100 g Gesamtmasse.

In einer möglichen Ausgestaltung der Erfindung erfolgt die Kristallisation durch Kühlung von Apparatewänden und/oder durch Verdampfung der Lösung im Vakuum. Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere Ausführungsform bei der Kühlungskristallisation ist die Verwendung von Kühlscheibenkristallern, wie sie z.B. von der Fa. Gouda (Holland) hergestellt werden. Bei einer weiteren geeigneten Variante zur Kristallisation durch Kühlung wird die Wärme über herkömmliche Wärmeüberträger (bevorzugt Rohrbündel oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den Wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit aufgeschmolzenen Kristallen). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen. In einer weiteren Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren (vgl. z.B. EP-A 616998) der Fa. Sulzer Chemtech (Schweiz) oder statisches Kristallisationsverfahren, (vgl. z.B. FR-A 2668946) der Fa. BEFS PROKEM (Frankreich)).

Die erhaltenen Acrylsäurekristalle werden wie bereits erwähnt von der verbliebenen Mutterlauge abgetrennt. Für den Fall der Schichtkristallisation oder der statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Beim Waschen liegt die Waschflüssigkeitsmenge geeigneterweise zwischen 0 und 500 g Waschflüssigkeit/100 g Kristallisat, vorzugsweise zwischen 30 und 200 g waschflüssigkeit/100 g Kristallisat. Die verwendete Waschflüssigkeit unterliegt keiner Einschränkung. Vorteilhafterweise wird jedoch mit Reinprodukt gewaschen, d.h. mit einer Flüssigkeit, die Acrylsäure enthält, deren Reinheit gleich oder höher ist als die des zu waschenden Kristallkuchens. Daneben ist auch eine Wäsche mit Wasser möglich. Das Waschen kann in hierfür üblichen Apparaten erfolgen.

Vorteilhafterweise werden Waschkolonnen (z.B. von der Fa. Niro Process Technology B.V., in s'Hertogenbusch (NL)) oder solche mit hydraulischem Betttransport (z.B. von der Firma TNO in Apeldoorn (NL))), in denen die Abtrennung der Mutterlauge und das waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge zwischen 0 und 80 g abgeschmolzenes Kristallisat/100 g Kristallisat vor dem Schwitzen, vorzugsweise zwischen 5 und 35 g abgeschmolzenes Kristallisat/100 g Kristallisat. Besonders bevorzugt ist die Durchführung des Schwitzens auf Zentrifugen oder Bandfiltern sowie in Kristallern, in denen die Kristalle im Kristaller fixiert sind (z.B. Schichtkristaller). Auch die Durchführung einer Kombination aus Waschen und Schwitzen in einem Apparat kann geeignet sein.

Die Acrylsäurekristalle nach der Fest-Flüssig-Trennung und ggf. weiterem Waschen und/oder Schwitzen stellen die gereinigte Säure aus dem Verfahren dar. Die Reinheit der erhaltenen Kristalle beträgt in der Regel 97 bis 99,99 Gew.-% und mehr Acrylsäure, insbesondere 98,5 bis 99,9 Gew.-% Acrylsäure. Die nach dem Verfahren hergestellten Kristalle enthalten nurmehr ganz geringe Mengen an Verunreinigungen, wie Essigsäure, Propionsäure und/oder Diacrylsäure.

Zusammenfassend sei nochmals festgehalten, daß die Kristallisation erfindungsgemäß prinzipiell als Suspensionskristallisation und/oder als Schichtkristallisation (die abgeschiedenen Kristalle verbleiben im Kristaller fixiert) realisiert werden kann. Als letztere Kristallisationsmethode kommt z.B. die Fallfilmkristallisation (z.B. wie in EP-A 616 998 beschrieben), die Kristallisation im volldurchströmten Rohr (z.B. gemäß DE-A 2606364) oder eine statische Kristallisation in Betracht.

Bei der Suspensionskristallisation kann die Kühlung direkt (z.B. Verdampfung im Vakuum) und/oder indirekt mittels gekühlter Flächen redisiert werden. Als Kristaller kommen für eine solche Suspensionskristallisation in Betracht: Rührkessel mit wandgängigen Rührern, Kratzkühler, Kühlscheibenkristaller der Fa. Gouda, sowie Umlaufkristallisation mit Wärmeüberträgern ohne Vorrichtung zur Vermeidung von Kristallschichtbildung.

Falls gewünscht, kann die gereinigte Säure nach bekannten Methoden verestert oder nach bekannten Methoden weiter gereinigt werden.

Zur Erhöhung der Ausbeute an Acrylsäure wird ganz generell die nach Beendigung der Kristallisation verbliebene Mutterlauge wenigstens teilweise, wie eingangs beschrieben, in die Trennkolonne rückgeführt. Der Anteil der rückgeführten Mutterlauge liegt, bezogen auf ihre anfallende Menge, erfindungsgemäß bei > 0 bis 100 Gew.-%, vorzugsweise bei 80 bis 100 Gew.-%. Über die Rückführung der Mutterlauge wird gleichzeitig die Rückführung des bei der Kristallisation abgetrennten Polymerisationsinhibitors gewährleistet. Dies garantiert eine voll befriedigende Polymerisationsinhibierung bei minimiertem Einsatz an Polymerisationsinhibitoren.

Bei Bedarf kann erfindungsgemäß zusätzlich zum Produktgemisch noch in begleitender Weise molekularer Sauerstoff oder ein molekularen Sauerstoff enthaltender Inertgasstrom durch die Trennkolonne geführt werden. Dies verstärkt die Wirkung der zugesetzten Polymerisationsinhibitoren.

In entsprechender Weise wie hier beschrieben, läßt sich selbstredend das zum beschriebenen Verfahren äquivalente Verfahren zur Herstellung von Methacrylsäure inhibieren und betreiben.

Mögliche Ausgangsverbindungen für die Gasphasenoxidation sind dabei Isobuten, Methyl-tert.butylether, Isobutan, Isobuttersäure, tert. Butanol, Isobutyraldehyd oder Methacrolein. Im übrigen gelten diesbezüglich die in der DE-A 19740253 und DE-A 19740252 gemachten Angaben.

Wie beschrieben, kann die Polymerisationsinhibierung beim erfindungsgemäßen Verfahren auch so vorgenommen werden, daß man am Kopf der Kolonne eine wäßrige MEHQ-Lösung aufgibt und in den Mittelteil der Kolonne eine Lösung von PTZ in Acrylsäure zusetzt. Der Sauerwasserquench wird dann ebenfalls mittels wäßriger MEHQ-Lösung stabilisiert.

### Beispiele

### Beispiel 1

(die in diesem Beispiel verwendeten Bezugsziffern beziehen sich auf die dieser Schrift beiliegende Figur)

Aus einer heterogenen katalysierten Gasphasenoxidation wurde ein eine Temperatur von 270°C aufweisendes Produktgasgemisch (1) der nachfolgenden Zusammensetzung erhalten:

| | |
|---|---|
| 11,5 Gew.-% | Acrylsäure, |
| 0,3 Gew.-% | Essigsäure, |
| 30 Gew.ppm | Propionsäure, |
| 0,09 Gew.-% | Maleinsäureanhydrid, |
| 0,01 Gew.-% | Acrolein, |
| 0,1 Gew.-% | Formaldehyd, |
| 30 Gew.-ppm | Furfural, |
| 0,001 Gew.-% | Benzaldehyd, |
| 0,3 Gew.-% | Propen, |
| 3,4 Gew.-% | Sauerstoff, |
| 5,3 Gew.-% | Wasser, |
| 1,7 Gew.-% | Kohlenoxide, und als Restmenge N₂. |

Das Produktgasgemisch (3600 g/h) wurde in einem Sprühkühler (2) auf eine Temperatur von 136°C abgekühlt. Als Sprühflüssigkeit wurden 750 g/h (7) von insgesamt 7000 g/h über den Fangboden (5) (mit einer Temperatur von 100°C) aus der Trennkolonne (3) entnommener Schwersiederfraktion (6) verwendet (Sumpfflüssigkeit 4 trat nicht auf). Über den mit Wärmeträgeröl beschriebenen Rohrbündelwärmetauscher (8) wurde die Sprühflüssigkeit im Kreis geführt. 40 g/h Schwersieder wurden dem Kreislauf kontinuierlich entnommen (9).

Das auf eine Temperatur von 136°C abgekühlte Produktgasgemisch wurde unterhalb des Fangbodens (5) der Trennkolonne zugeführt (10).

Die Kolonne war eine Bodenkolonne mit, von unten nach oben betrachtet, zunächst 25 Dual-Flow- und anschließend 50 Glockenböden (1 Glocke pro Boden). Der Bodendurchmesser betrug 49 mm. Die Dual-Flow-Böden wiesen 6 Löcher pro Boden auf. Der Lochdurchmesser der ersten fünf Dual-Flow-Böden betrug 9,5 mm. Die darauffolgenden 10 Böden hatten einen Lochdurchmesser von 9 mm und der Lochdurchmesser der letzten 5 Dual-Flow-Böden betrug 8,7 mm. Der Boden oberhalb von Boden 15 war als weiterer Fangboden (11) gestaltet. Über ihm wurden 1800 g/h einer eine Temperatur von 97°C aufweisenden Rohacrylsäure (12) enthaltend

| | |
|---|---|
| Acrylsäure | 97,3 Gew.-% |
| Essigsäure | 0,8 Gew.-% |
| Propionsäure | 600 Gew.ppm |
| Furfural | 700 Gew.ppm |
| Maleinsäureanhydrid | 40 Gew.ppm |
| Benzaldehyd | 200 Gew.-ppm |
| Wasser | 1,3 Gew.-% |

abgezogen und einem Suspensionskristaller (13) zugeführt. Ein Teil (6250 g/h) der entnommenen Schwersiederfraktion (14) wurde in einem mit Wärmeträgeröl beschriebenen Rohrbündelwärmetauscher auf 105°C erwärmt und auf den 5ten Boden in die Kolonne rückgeführt (16).

Der Kristaller war ein Rührbehälter (3 l Innenvolumen) mit Wendelrührer. Die Kristallisationswärme wurde über den Doppelmantel des Behälters abgeführt. Die Gleichgewichtstemperatur der Lösung betrug 9,7°C. Die bei der Kristallisation erzeugte Suspension (Feststoffgehalt ca. 25 Gew.-%) wurde auf einer Zentrifuge bei 2000 U/min (Zentrifugendurchmesser 300 mm) und einer Schleuderzeit von 3 min diskontinuierlich in Kristalle und Mutterlauge getrennt. Die Kristalle wurden anschließend mit aufgeschmolzenem (zuvor gewaschenem) Kristallisat (80 g) 20 sec lang bei 2000 U/min gewaschen. Die Mutterlauge wurde zusammen mit der waschflüssigkeit auf den 15ten Boden in die Trennkolonne rückgeführt (28).

Die Analyse der Kristalle (370 g/h) ergab folgende Gehalte:

| | |
|---|---|
| Acrylsäure | 99,5 Gew.-% |
| Essigsäure | 0,2 Gew.-% |
| Propionsäure | 200 Gew.ppm |
| Maleinsäureanhydrid | 20 Gew.ppm |
| Furfural | 20 Gew.ppm |
| Benzaldehyd | 5 Gew.-ppm |
| Wasser | 0,06 Gew.-% |

Am Kopf der Kolonne wurde ein gasförmiges Gemisch (17) entnommen und im Sprühkühler (18) einer Partialkondensation unterworfen. 480 g/h des dabei anfallenden Sauerwassers wurden am Kopf der Kolonne mit einer Temperatur von 30°C in selbige zurückgeführt (26). 220 g/h des Sauerwassers wurden kontinuierlich entnommen (das Sauerwasser enthielt 3 Gew.-% Acrylsäure und 2,6 Gew.-% Essigsäure). 90 g/h des entnommenen Sauerwassers wurden mit MEHQ (22) versetzt und als 0,5 gew.-%ige wässrige Stabilisatorlösung (21) gemeinsam mit der Restmenge des Sauerwassers (23) über den wassergekühlten Rohrbündelwärmetauscher (24) auf 18°C abgekühlt als Sprühflüssigkeit (25) verwendet. Mit einem anderen Teil des entnommenen Sauerwassers wurde eine 0,5 gew.-%ige wäßrige Lösung von 4-Hydroxy-TEMPO hergestellt, die in einer Menge von 18 g/h mit einer Temperatur auf dem 75ten Boden der Trennkolonne zugeführt wurde (27).

Die beschriebene Trennvorrichtung konnte 40 Tage ohne nennenswerte Polymerisatbildung betrieben werden.

### Vergleichsbeipsiel 1

(die in diesem Beispiel verwendeten Bezugsziffern beziehen sich auf die dieser Schrift beiliegenden Figur)

Aus einer heterogen katalysierten Gasphasenoxidation wurde ein eine Temperatur von 270°C aufweisendes Produktgasgemisch (1) der nachfolgenden Zusammensetzung erhalten:

| | |
|---|---|
| 11,5 Gew.-% | Acrylsäure, |
| 0,3 Gew.-% | Essigsäure, |
| 30 Gew.ppm | Propionsäure, |
| 0,09 Gew.-% | Maleinsäureanhydrid, |
| 0,1 Gew.-% | Acrolein, |
| 0,1 Gew.-% | Formaldehyd, |
| 30 Gew.-ppm | Furfural, |
| 0,01 Gew.-% | Benzaldehyd, |
| 0,3 Gew.-% | Propen, |
| 3,4 Gew.-% | Sauerstoff, |
| 5,3 Gew.-% | Wasser, |
| 1,7 Gew.-% | Kohlenoxide, und als Restmenge N₂ |

Das Produktgasgemisch (3600 g/h) wurde in einem Sprühkühler (2) auf eine Temperatur von 136°C abgekühlt. Als Sprühflüssigkeit wurden 750 g/h (7) von insgesamt 7000 g/h über den Fangboden (5) (mit einer Temperatur von 100°C) aus der Trennkolonne (3) entnommener Schwersiederfraktion (6) verwendet (Sumpfflüssigkeit 4 wurde nicht mitverwendet). Über den mit Wärmeträgeröl betriebenen Rohrbündelwärmetauscher (8) wurde die Sprühflüssigkeit im Kreis geführt. 40 g/h Schwersieder wurden dem Kreislauf kontinuierlich entnommen (9).

Das auf eine Temperatur von 136°C abgekühlte Produktgasgemisch wurde unterhalb des Fangbodens (5) der Trennkolonne zugeführt (10).

Die Kolonne war eine Bodenkolonne mit 75 Dual-Flow-Böden. Der Boden oberhalb von Boden 15 war als weiterer Fangboden (11) gestaltet. Über ihm wurden 1620 g/h einer eine Temperatur von 92°C aufweisenden Rohacrylsäure (12) enthaltend

| | |
|---|---|
| Acrylsäure | 96,6 Gew.-% |
| Essigsäure | 1,9 Gew.-% |
| Propionsäure | 430 Gew.ppm |
| Furfural | 470 Gew.ppm |
| Maleinsäureanhydrid | 40 Gew.ppm |
| Benzaldehyd | 300 Gew.-ppm |
| Wasser | 1,2 Gew.-% |

abgezogen und einem Suspensionskristaller (13) zugeführt. Ein Teil (7400 g/h) der entnommenen Schwersiederfraktion (14) wurde in einem mit Wärmeträgeröl beschriebenen Rohrbündelwärmetauscher auf 100°C erwärmt und auf den Sten Boden in die Kolonne rückgeführt (16).

Der Kristaller war ein Rührbehälter (3 l Innenvolumen) mit Wendelrührer. Die Kristallisationswärme wurde über den Doppelmantel des Behälters abgeführt. Die Gleichgewichtstemperatur der Lösung betrug 9,7°C. Die bei der Kristallisation erzeugte Suspension (Feststoffgehalt ca. 22 Gew.-%) wurde auf einer Zentrifuge bei 2000 U/min (Zentrifugendurchmesser 300 mm) und einer Schleuderzeit von 3 min diskontinuierlich in Kristalle und Mutterlauge getrennt. Die Kristalle (360 g/h) wurden anschließend mit aufgeschmolzenem (zuvor gewaschenem) Kristallisat (80 g) 20 sec lang bei 2000 U/min gewaschen. Die Mutterlauge wurde zusammen mit der Waschflüssigkeit auf den 15ten Boden in die Trennkolonne rückgeführt (28).

Die Analyse der Kristalle (330 g/h) ergab folgende Gehalte:

| | |
|---|---|
| Acrylsäure | 99,0 Gew.-% |
| Essigsäure | 0,5 Gew.-% |
| Propionsäure | 160 Gew.ppm |
| Maleinsäureanhydrid | 20 Gew.ppm |
| Furfural | 60 Gew.ppm |
| Benzaldehyd | 30 Gew.-ppm |
| Wasser | 0,25 Gew.-% |

Am Kopf der Kolonne wurde ein gasförmiges Gemisch (17) entnommen und im Sprühkühler (18) einer Partialkondensation unterworfen. 690 g/h des dabei anfallenden Sauerwassers wurden am Kopf der Kolonne mit einer Temperatur von 30°C in selbige zurückgeführt (26). 270 g/h des Sauerwassers wurden kontinuierlich entnommen (das Sauerwasser enthielt 7,9 Gew.-% Acrylsäure und 0,84 Gew.-% Essigsäure). 90 g/h des entnommenen Sauerwassers wurden mit MEHQ (22) versetzt und als 0,5 gew.-%ige wässrige Stabilisatorlösung (21) gemeinsam mit der Restmenge des Sauerwassers (23) über den wassergekühlten Rohrbündelwärmetauscher (24) auf 18°C abgekühlt als Sprühflüssigkeit (25) verwendet. Mit einem anderen Teil des entnommenen Sauerwassers wurde eine 0,5 gew.-%ige wäßrige Lösung von 4-Hydroxy-TEMPO hergestellt, die in einer Menge von 18 g/h mit einer Temperatur auf dem 75ten Boden der Trennkolonne zugeführt wurde (27).

### Vergleichsbeispiel 2

Es wurde wie in Vergleichsbeispiel 1 verfahren. Die Kolonne für die fraktionierte Kondensation war jedoch eine Bodenkolonne mit 75 Glockenböden. Nach 26 Tage mußte der Betrieb der beschriebenen Trennvorrichtung infolge Blokade durch Polymerisat unterbrochen werden.

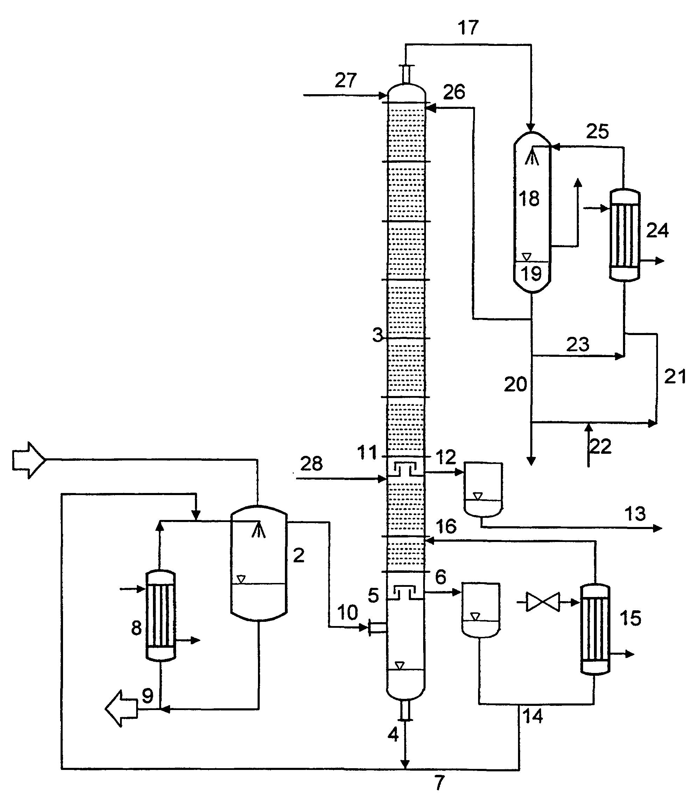

## Patentansprüche

1. Verfahren der fraktionierten Kondensation eines Acrylsäure enthaltenden Produktgasgemisches einer heterogen katalysierten Gasphasen-Partialoxidation von C₃-Vorläufern der Acrylsäure mit molekularem Sauerstoff in einer hydraulisch abgedichtete Querstromböden als trennwirksame Einbauten enthaltenden Trennkolonne, **dadurch gekennzeichnet, daß** als Trennkolonne eine solche verwendet wird, die von unten nach oben zunächst Dual-Flow Böden und im Anschluß daran hydraulisch abgedichtete Querstromböden als trennwirksame Einbauten enthält.

2. Trennkolonne, die als trennwirksame Einbauten von unten nach oben zunächst Dual-Flow Böden und im Anschluß daran hydraulisch abgedichtete Querstromböden enthält.

## Claims

1. A process for the fractional condensation of an acrylic acid-containing product gas mixture of a gas-phase partial oxidation of C₃ precursors of acrylic acid with molecular oxygen under heterogeneous catalysis in a separation column containing hydraulically sealed cross-flow trays as baffles having separation activity, wherein the separation column used is one which contains, from bottom to top, first dual-flow trays and then hydraulically sealed cross-flow trays as baffles having separation activity.

2. A separation column which contains, from bottom to top, first dual-flow trays and then hydraulically sealed cross-flow trays as baffles having separation activity.

## Revendications

1. Procédé de condensation fractionnée d'un mélange de produits gazeux contenant de l'acide acrylique, provenant d'une oxydation partielle en phase gazeuse à catalyse hétérogène de précurseurs en C₃ de l'acide acrylique par de l'oxygène moléculaire, dans une colonne à plateaux à courant transversal hydrauliquement étanches en tant qu'éléments encastrés à action de séparation, **caractérisé en ce que** l'on utilise comme colonne de séparation une colonne comportant comme éléments encastrés séparateurs des plateaux dualflow d'abord, et ensuite, de bas en haut, des plateaux à courant transversal hydrauliquement étanches.

2. Colonne de séparation, comportant comme éléments encastrés séparateurs des plateaux dualflow d'abord, et ensuite, de bas en haut, des plateaux à courant transversal hydrauliquement étanches.
